# FASCICULE DE BREVET EUROPEEN

(11) **EP 3 218 035 B1**
(45) Date de publication et mention de la délivrance du brevet: **22.05.2019**
(21) Numéro de dépôt: 16710259.9
(22) Date de dépôt: 26.02.2016
(51) Int. Cl.: A61M 16/10, A61M 16/00

(54) **APPAREIL DE VENTILATION ARTIFICIELLE APTE À DÉLIVRER UNE VENTILATION ET UN MONITORAGE SPÉCIFIQUES AUX PATIENTS RECEVANT UN MASSAGE CARDIAQUE**
VORRICHTUNG ZUR KÜNSTLICHEN BEATMUNG FÜR DIE SPEZIFISCHE BEATMUNG UND ÜBERWACHUNG VON PATIENTEN UNTER HERZMASSAGE
ARTIFICIAL VENTILATION APPARATUS ABLE TO DELIVER VENTIALTION AND MONITORING WHICH ARE SPECIFIC TO THE PATIENTS RECEIVING CARDIAC MASSAGE

(30) Priorité: 28.04.2015 FR 1553808
(43) Date de publication de la demande: 20.09.2017
(60) Demande divisionnaire: 17196732.6
(73) Titulaire: Air Liquide Medical Systems, 92160 Antony (FR)
(72) Inventeur: JACQUOT, Eric, 92160 Antony (FR); RIGOLLOT, Marceau, 92120 Montrouge (FR); RICHARD, Jean-Christophe, 92160 Antony (FR)
(74) Mandataire: Pittis, Olivier
(86) Numéro de dépôt international: PCT/FR2016/050445
(87) Numéro de publication internationale: WO 2016/174318

(56) Documents cités:
- EP-A1- 1 121 952
- FR-A1- 2 987 274
- FR-A1- 3 000 893

## Description

L'invention concerne un appareil de ventilation artificielle permettant d'apporter une assistance ventilatoire, en particulier ventilation et monitorage, à un secouriste ou tout autre personnel soignant, tel un médecin du SAMU, un pompier, un infirmier ou analogue, lorsque celui-ci pratique un massage cardiaque.

Il est usuel d'utiliser un appareil de ventilation artificielle, encore appelé appareil d'assistance respiratoire ou ventilatoire, ou plus simplement ventilateur médical, pour apporter une assistance respiratoire, c'est-à-dire une ventilation artificielle, à une personne ayant des difficultés ou une incapacité à respirer seule.

En particulier, il est indispensable de ventiler une personne pendant un arrêt cardiaque afin de continuer à apporter de l'oxygène à son cerveau et au reste du corps, alors que son coeur est arrêté.

Cependant, opérer une ventilation sur une personne en arrêt cardiaque, alors qu'un massage cardiaque est pratiqué sur cette personne, est problématique car cette ventilation ne doit pas interrompre les compressions thoraciques (CT) et/ou ne doit pas être excessive pour ne pas avoir d'effets hémodynamiques négatifs.

Or, les ventilateurs conventionnels ne sont pas adaptés à cette situation, émettent des alarmes et/ou dysfonctionnent pendant les compressions thoraciques.

Dès lors, en pratique, les personnels soignants délivrent les insufflations, via un ventilateur ou un ballon auto-remplisseur à valve unidirectionnelle (BAVU) en interrompant parfois les compressions thoraciques.

La continuité et la régularité du massage cardiaque est un facteur d'efficacité sur le pronostic du patient en arrêt cardiaque. Il n'est donc nullement conseillé de stopper un massage cardiaque, même pour opérer des insufflations de gaz respiratoire au patient.

En outre, les insufflations délivrées manuellement, via un BAVU, ou par un ventilateur conventionnel sont le plus souvent trop agressives, notamment du fait de grands volumes de gaz administrés, entrainant un effet direct délétère et reconnu sur l'efficacité des compressions thoraciques.

Pour cette raison, des appareils évitant les insufflations classiques, notamment des ventilateurs à pression positive continue de type CPAP (Continuous Positive Airway Pressure) ont été proposés en vue d'être utilisés en cas d'arrêt cardiaque. Cependant, ces appareils ne sont pas idéaux car ils ne permettent pas d'assurer une ventilation suffisante de la personne en arrêt cardiaque et l'arrêt des compressions engendre un arrêt de toute ventilation, ce qui n'est pas souhaitable.

Par ailleurs, la plupart des appareils capables de délivrer une ventilation mécanique pendant un massage cardiaque ne permettent pas d'assurer cette ventilation de façon autonome/automatique, alors que cette situation clinique complexe nécessite une grande simplicité d'utilisation. En effet, ces appareils requièrent souvent une intervention humaine lors de chaque insufflation, ce qui complique l'intervention du personnel soignant et conduit parfois à une administration de gaz à une pression et/ou à un volume mal maîtrisés.

Or, si pendant une insufflation de gaz respiratoire, la pression et le volume générés ne sont pas correctement maitrisés, il peut se produire des phénomènes néfastes au patient, comme une insufflation gastrique indésirable.

En d'autres termes, avec les appareils classiques, il n'est pas possible de délivrer de façon sûre et simple, une ventilation protectrice qui ne dépasse pas le volume de gaz recommandé.

En outre, au cours du massage cardiaque les compressions de la cage thoracique génèrent une ventilation pulmonaire substantielle mais insuffisante, laquelle doit être complétée par une ventilation mécanique. Quand les compressions thoraciques sont interrompues, après reprise d'activité circulatoire spontanée du patient ou après un choc électrique par exemple, la ventilation diminue brutalement, alors que les besoins en oxygène du malade augmentent. Un complément de ventilation est alors nécessaire imposant des manipulations complexes pour changer de mode de ventilation afin de pouvoir palier ce manque d'oxygène.

Ensuite, une fois ce changement de ventilation opéré et le patient ventilé de façon adaptée, un nouvel arrêt cardiaque peut survenir. Dans ce cas, les compressions thoraciques sont reprises et il est alors à nouveau nécessaire de modifier manuellement les réglages du dispositif de ventilation pour se repositionner dans le mode de ventilation le plus adapté pour opérer une réanimation cardio-pulmonaire (RCP).

Sachant qu'une personne peut connaître plusieurs arrêts cardiaques successifs pendant sa prise en charge par une équipe médicale, délivrer une ventilation optimale est compliqué car cela nécessite une intervention de l'équipe médicale, lors de la survenue de chacun de ces arrêts cardiaques, pour adapter l'appareil. Or, en situation d'urgence, le temps consacré à ces adaptations est au détriment d'opérations plus importantes. Faute de temps, ces adaptations peuvent être oubliées, ignorées ou négligées, ce qui n'est pas acceptable pour des questions évidentes de sécurité.

De façon générale, la plupart des appareils de ventilation mécanique connus susceptibles d'être utilisés pendant un massage cardiaque, ne disposent pas d'un mode spécifique adapté à cette situation. Ils sont simplement équipés de déclencheurs inspiratoires qui s'activent souvent à tort et provoquent des auto-déclenchements et des cycles néfastes au débit sanguin cardiaque provoqué par les CT. De plus, certains n'offrent pas la possibilité de régler une pression expiratoire positive (PEEP) pourtant indispensable.

Enfin, de nombreuses alarmes sonores et/ou visuelles équipant ces appareils classiques s'activent également à tort, par exemple des alarmes de pression, de volume ou de fréquence car elles sont développées pour des applications « classiques » et que les déclencheurs et les alarmes utilisent les signaux de pression et de débit mesurés par la machine, qui sont, quant à eux, très perturbés par le massage cardiaque.

Le document FR-A-3000893 décrit un appareil de ventilation artificielle, conforme au préambule de la revendication 1, comprenant des moyens de traitement de signal permettant d'analyser un signal de type débit, pression ou vitesse de turbine pour en déduire la réalisation d'un massage cardiaque et la fréquence dudit massage, laquelle peut être affichée ensuite.

Le problème qui se pose est dès lors de proposer un appareil de ventilation artificielle, c'est-à-dire d'assistance respiratoire, encore appelé ventilateur médical, permettant de résoudre tout ou partie des problèmes et inconvénients susmentionnés, en particulier un ventilateur médical permettant de détecter la réalisation d'un massage cardiaque sur le patient en arrêt cardiaque, en particulier des phases de compression et de décompression, dans le but de transmettre au coeur un maximum d'énergie mécanique engendrée par les CT en limitant l'énergie dissipée via les voies aériennes, y compris les poumons. Le ventilateur doit également être capable de délivrer une ventilation barométrique à deux niveau de pression sur laquelle peuvent se superposer les CT.

La solution de l'invention concerne alors un appareil d'assistance respiratoire, encore appelé ventilateur médical, comprenant :
- une micro-soufflante motorisée ou un dispositif d'alimentation en gaz comprenant une valve inspiratoire en tant que source de gaz, et
- un circuit de gaz avec au moins une branche inspiratoire apte à véhiculer un gaz respiratoire destiné à être administré à un patient en arrêt cardiaque pendant une réanimation cardio-pulmonaire,
- des moyens de mesure aptes à et conçus pour :
   i) mesurer au moins un paramètre représentatif dudit flux de gaz,
   ii) convertir ledit au moins un paramètre représentatif dudit flux de gaz en au moins un signal représentatif dudit flux de gaz,
- et des moyens de traitement de signal et de pilotage aptes à et conçus pour :
   a) traiter ledit au moins un signal représentatif du flux de gaz fourni par les moyens de mesure et
   b) déduire dudit au moins signal représentatif du flux de gaz, une information relative à la réalisation d'un massage cardiaque sur le patient en arrêt cardiaque,
caractérisé en ce que les moyens de traitement de signal et de pilotage sont configurés pour piloter la micro-soufflante motorisée ou la valve inspiratoire de manière à :
i) en réponse à la détection d'une phase de compression, augmenter le volume ou la pression de gaz fourni par la micro-soufflante motorisée ou la valve inspiratoire, et
ii) en réponse à la détection d'une phase de relâchement/décompression, diminuer le volume ou la pression de gaz fourni par la micro-soufflante motorisée ou la valve inspiratoire.

Selon le cas, l'appareil de l'invention peut comprendre l'une ou plusieurs des caractéristiques techniques suivantes :
- les moyens de traitement de signal et de pilotage sont aptes à et conçus pour transmettre des signaux de consigne aux actionneurs, c'est-à-dire typiquement à la source de gaz, par exemple une microsoufflante, ou à une ou des électrovannes pilotées par lesdits moyens de traitement de signal et de pilotage.
- les moyens de traitement de signal et de pilotage sont aptes à et conçus pour déduire dudit au moins un signal représentatif du flux de gaz, au moins une information relative à au moins une phase de compression et/ou de relâchement/décompression de la cage thoracique lors d'un massage cardiaque opéré sur le patient.
- les moyens de traitement de signal et de pilotage sont aptes à et conçus pour déduire dudit au moins un signal représentatif du flux de gaz, des informations relatives à une alternance de phases de compression et/ou de relâchement/décompression de la cage thoracique lors d'un massage cardiaque opéré sur le patient.
- les moyens de traitement de signal et de pilotage comprennent une carte électronique.
- la source de gaz comprend une micro-soufflante motorisée, encore appelée turbine ou compresseur, à moteur électrique.
- la source de gaz est une micro-soufflante motorisée pilotée par les moyens de traitement de signal et de pilotage, ladite micro-soufflante motorisée étant en communication fluidique avec la branche inspiratoire du circuit de gaz.
- alternativement, le dispositif d'alimentation en gaz comprend une valve inspiratoire agencée sur un conduit d'alimentation en gaz, ladite valve inspiratoire étant pilotée par les moyens de traitement de signal et de pilotage. Dans ce cas, la source de gaz est extérieure à l'appareil mais est en communication fluidique avec ledit conduit d'alimentation en gaz sur lequel est agencée la valve inspiratoire pilotée, par exemple il s'agit d'une prise murale alimentée en gaz par une canalisation de gaz au sein d'un bâtiment hospitalier par exemple et à laquelle vient se raccorder fluidiquement à l'appareil de l'invention via un conduit flexible par exemple.
- il comprend un moyen de sélection, permettant de sélectionner un mode ventilatoire donné spécifique à une réanimation cardio-pulmonaire parmi plusieurs modes ventilatoires mémorisés. La sélection de ce mode permet de démarrer des surveillances, monitorages et régulations spécifiques à la ventilation pendant la réanimation cardio-pulmonaire. Le lancement de ce mode permet également de commencer la ventilation avec des réglages prédéfinis et préalablement enregistrés par l'utilisateur.
- les moyens de mesure conçus pour et aptes à mesurer au moins un paramètre représentatif du flux de gaz choisi parmi la pression du gaz, le débit de gaz insufflé au patient, le débit de gaz expiré par le patient et la vitesse de la micro-soufflante.
- au moins une partie du circuit de gaz, les moyens de traitement de signal et la micro-soufflante motorisée ou la valve inspiratoire du dispositif d'alimentation sont situés dans une carcasse rigide, c'est-à-dire une enveloppe externe formant la coque ou le capotage de l'appareil.
- le circuit de gaz comprend une portion interne agencée dans la carcasse rigide et une portion externe située hors de la carcasse rigide et formant tout ou partie de la branche inspiratoire.
- il comprend en outre une interface homme-machine apte à afficher des informations incluant au moins une information relative à la réalisation d'un massage cardiaque sur le patient en arrêt cardiaque.
- le circuit de gaz comprend en outre une branche expiratoire en communication fluidique avec l'atmosphère via un orifice de sortie de gaz, et comportant une valve expiratoire et un capteur de débit expiratoire.
- le capteur de débit expiratoire est préférentiellement un capteur à fil chaud.
- la portion externe de la branche inspiratoire du circuit de gaz est en communication fluidique avec une interface respiratoire, en particulier un masque respiratoire ou une canule d'intubation.
- le capteur de débit expiratoire est agencé entre la valve expiratoire et l'orifice de sortie de gaz communiquant avec l'atmosphère.
- de façon alternative, le capteur de débit expiratoire est agencé entre la valve expiratoire et l'interface respiratoire alimentant le patient en gaz.
- les moyens de traitement de signal et de pilotage sont configurés pour piloter la micro-soufflante motorisée ou la valve inspiratoire, et la valve expiratoire en fonction de signaux reçus des moyens de mesure et du capteur de débit expiratoire.
- les moyens de traitement de signal et de pilotage sont configurés pour, c'est-à-dire conçus pour et aptes à, piloter la micro-soufflante motorisée ou la valve inspiratoire de la source de gaz de manière à augmenter le volume ou la pression de gaz fourni par la micro-soufflante motorisée ou la valve inspiratoire en réponse à la détection d'une phase de compression, dans le but d'amplifier le pic de pression provoqué par la compression thoracique du patient en freinant l'éjection d'un volume d'air contenu dans le système respiratoire dudit patient.
- les moyens de traitement de signal et de pilotage sont configurés pour, c'est-à-dire conçus pour et aptes à, piloter la micro-soufflante motorisée ou la valve inspiratoire de la source de gaz de manière à diminuer le volume ou la pression de gaz fourni par la micro-soufflante motorisée ou la valve inspiratoire en réponse à la détection d'une phase de relâchement/décompression de manière à favoriser un retour passif de la cage thoracique du patient en position d'équilibre.
- les moyens de traitement de signal et de pilotage sont configurés pour piloter la valve expiratoire de manière à limiter ou stopper le débit de gaz traversant la valve expiratoire en réponse à la détection d'une phase de compression ou de relâchement/décompression.
- les moyens de traitement de signal et de pilotage comprennent au moins un microprocesseur mettant en oeuvre au moins un algorithme.
- il comprend en outre des moyens de stockage de données, c'est-à-dire des moyens de mémorisation.
- les moyens de stockage de données comprennent une (ou plusieurs) mémoire, en particulier une mémoire flash ou analogue.
- les moyens de stockage de données sont configurés pour mémoriser plusieurs modes ventilatoires comprenant un (ou plusieurs) mode ventilatoire donné spécifique à une réanimation cardio-pulmonaire.
- les moyens de mesure sont conçus pour et aptes à mesurer au moins un paramètre représentatif du flux de gaz choisi parmi la pression du gaz, le débit de gaz insufflé au patient, le débit de gaz expiré par le patient et la vitesse de la micro-soufflante.
- la portion interne du circuit de gaz est en communication fluidique avec la source de gaz, en particulier une micro-soufflante, de manière à être alimentée avec du gaz délivré par ladite source de gaz.
- les moyens de mesure sont agencés sur le circuit de gaz à l'intérieur ou à l'extérieur de la carcasse rigide de manière à y opérer les mesures souhaitées.
- il comprend plusieurs moyens de mesure dont certains sont agencés sur la portion interne du circuit de gaz et d'autres sur la portion externe du circuit de gaz.
- les moyens de mesure comprennent un ou des capteurs.
- les moyens de mesure comprennent un capteur agencé de manière à opérer des mesures sur dans le circuit de gaz, de préférence dans la branche inspiratoire du circuit de gaz.
- il comprend en outre une interface homme-machine (IHM) apte à, c'est-à-dire conçue pour, afficher des informations incluant au moins une information relative à la réalisation d'un massage cardiaque sur le patient en arrêt cardiaque.
- l'interface homme-machine comprend un écran d'affichage.
- la branche inspiratoire du circuit de gaz est en communication fluidique avec une interface respiratoire, en particulier un masque respiratoire ou une canule d'intubation.
- la branche inspiratoire du circuit de gaz comprend un tube ou conduit flexible.
- la source de gaz est une source d'air (env. 21 vol. % d'O₂) ou d'air enrichi en oxygène (>21 vol.% d'O₂ environ)
- les moyens de traitement de signal comprennent au moins un microprocesseur, de préférence agencé sur une carte électronique.
- les moyens de traitement de signal comprennent au moins un microcontrôleur, de préférence au moins un microcontrôleur mettant en oeuvre au moins un algorithme.
- la carcasse comprend au moins une poignée de portage facilitant le transport de l'appareil par un utilisateur.
- la carcasse comprend au moins un dispositif d'accrochage permettant l'accroche de l'appareil de ventilation à un support, par exemple une tringle au sein d'un véhicule d'urgence ou un barreau de lit ou de brancard.
- il comprend des moyens d'alimentation en courant électrique, par exemple une (ou plusieurs) batterie ou analogue, ou un (ou plusieurs) câble et une (ou plusieurs) prise de raccordement au secteur électrique.
- il comporte en outre des moyens de réglage et de sélection, tel que bouton-poussoir, touche d'activation, curseur ou analogue, permettant au personnel soignant d'intervenir au niveau du ventilateur, par exemple d'indiquer au ventilateur, la réalisation d'un massage cardiaque, de lui confirmer une détection d'un massage cardiaque, de lui indiquer le type d'interface respiratoire utilisée (masque, sonde d'intubation...), de modifier un ou des paramètres de ventilation mécanique proposés automatiquement par le ventilateur, ou tout autre indication.

De façon générale, concernant le mode de ventilation spécifique à la réanimation cardio-pulmonaire, celui-ci peut être un mode volumétrique ou barométrique, de préférence associé à une pression minimale de ventilation, par exemple 5 cm d'H₂O.

Avantageusement, il s'agit d'un mode barométrique qui assure une régulation alternée sur 2 niveaux de pression comprenant un niveau de pression basse (PB) et un niveau de pression haute (PH), avec PH>PB, par exemple une pression basse de l'ordre de 5 cm d'H₂O, et une pression haute de l'ordre de 15 cm d'H₂O.

Le mode de ventilation spécifique à la réanimation cardio-pulmonaire permet d'assurer la ventilation d'un patient du début à la fin de l'intervention dans un environnement nécessitant peu ou pas d'interventions humaines, lors des différentes phases.

L'appareil d'assistance respiratoire de l'invention dispose, outre ce mode de ventilation spécifique à la réanimation cardio-pulmonaire, d'autres modes de ventilation conventionnelle comme un ou des modes de ventilation volumétriques (VAC), barométriques (VPC, VSAI, CPAP, Duo-Levels..) et/ou intermittents (VACI, PVACI).

Pour améliorer la circulation sanguine à partir de la ventilation, l'appareil de l'invention est configuré pour moduler l'ouverture de la valve expiratoire et l'accélération de la micro-soufflante, pendant le temps de compression thoracique, de manière à limiter la dissipation d'énergie en freinant l'éjection du volume d'air contenu dans le système respiratoire, afin d'amplifier la pression transmise au coeur.

Pendant le temps de décompression ou remonté de la cage thoracique, l'appareil de l'invention est configuré pour moduler la vitesse de la micro-soufflante, de manière à favoriser un retour passif de la cage thoracique en position d'équilibre afin que la pression intrathoracique reste négative permettant ainsi le retour veineux et le remplissage du coeur.

La succession, par exemple à une fréquence de 100 fois par minute, des phases de compression et de décompression permet, en alternant successivement pression positive et négative, de générer du débit cardiaque par un effet pompe. En d'autres termes, en modulant la vitesse de la micro-soufflante, au rythme des CT et l'ouverture de la valve expiratoire, l'appareil selon l'invention joue le rôle d'un amplificateur des variations de pression générées par les CT, par opposition à un appareil cherchant à réguler une pression constante à un ou deux niveaux de pression. Exemple CPAP ou mode PAC.

En pratique, l'appareil opère une modulation, à la fréquence du massage cardiaque, de la vitesse de la micro-soufflante et de l'ouverture de la valve expiratoire, contrairement à un mode classique en pression.

En effet, avec une CPAP ou un mode en pression classique, le principe est de maintenir la pression la plus constante possible quelles que soient les perturbations, notamment les CT de la RCP. Or, ce fonctionnement classique ne permet pas une optimisation des pressions générées par les CT qui sont la clef de l'efficacité de la RCP.

Enfin, pour compléter la ventilation qui est insuffisante en cas de CT seules, l'appareil de l'invention est configuré pour permettre d'améliorer/accroitre la ventilation délivrée par le ventilateur par l'adjonction de cycles en pression (comme en pression contrôlée) à la fréquence choisie. Les CT sur ces cycles restent possibles, ce qui distingue l'appareil de l'invention d'un appareil classique fonctionnant selon un mode en pression ou en volume.

Il est précisé que, dans le cadre de la présente invention, le terme « moyens » est considéré comme étant strictement équivalent du terme « dispositif ». De là, les termes « moyens de mesure » équivalent à « dispositif de mesure »; les termes « moyens d'affichage » équivalent à « dispositif d'affichage »; les termes « moyens de traitement » équivalent à « dispositif de traitement »; les termes « moyens de stockage de données » équivalent à « dispositif de stockage de données »; etc...

La présente invention va maintenant être décrite plus en détail en références aux Figures annexées parmi lesquelles:
- la Figure 1 représente un premier mode de réalisation d'un appareil d'assistance respiratoire selon la présente invention, dans lequel la source de gaz est une micro-soufflante,
- la Figure 2 représente un second mode de réalisation d'un appareil d'assistance respiratoire selon la présente invention, dans lequel la source de gaz est un dispositif d'alimentation comprenant une valve pilotée, et
- la Figure 3 est une représentation graphique de la pression intra-thoracique obtenue grâce à un appareil d'assistance respiratoire selon la présente invention

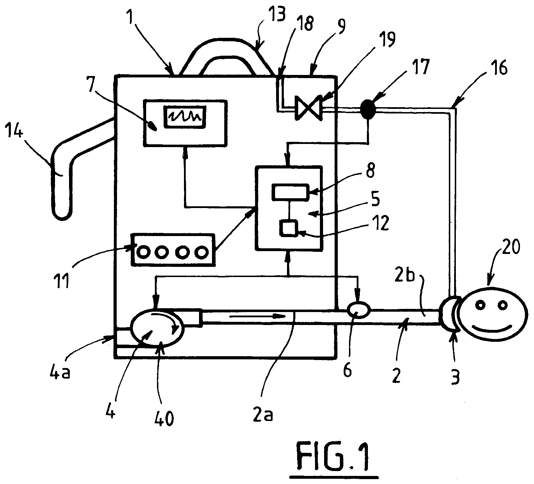

La Figure 1 schématise un premier mode de réalisation d'un appareil d'assistance ventilatoire ou ventilateur médical 1 selon la présente invention.

Le ventilateur 1 comprend une source de gaz 4, à savoir ici une micro-soufflante motorisée 40, encore appelée turbine, délivrant un flux de gaz d'assistance respiratoire, typiquement un flux d'air ou d'air enrichi en oxygène, dans la branche inspiratoire 2 d'un circuit ventilatoire 2, 16, encore appelé circuit patient, comprenant un ou plusieurs passages, conduits ou lignes de gaz, reliant fluidiquement le ventilateur 1 aux voies aériennes d'un patient 20, par l'intermédiaire d'une interface patient 3, tel un masque respiratoire ou une canule ou sonde d'intubation.

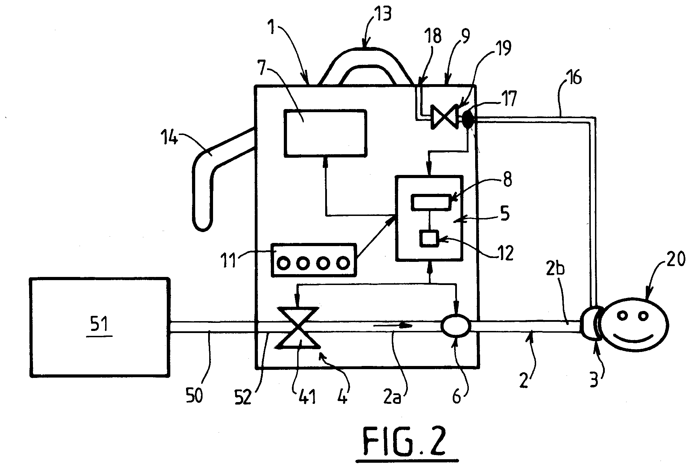

De manière alternative, comme illustré dans le second mode de réalisation de la Figure 2, la source de gaz 4 peut comprendre une valve inspiratoire 41 alimentée par du gaz provenant d'une canalisation de gaz 50, elle-même alimentée par un stockage de gaz 51, par exemple un réseau hospitalier de canalisations de gaz, une bouteille ou un compresseur de gaz externe. La canalisation de gaz 50 est en communication fluidique avec un conduit de gaz interne 52 au ventilateur 1, sur lequel est agencée la vanne inspiratoire 41.

Le gaz respiratoire est typiquement de l'air ou de l'oxygène, à une pression de quelques bars absolus, typiquement de l'ordre de 4 bar abs environ. Cette valve inspiratoire est pilotée par les moyens de traitement de signal et de pilotage 5, 8 de l'appareil 1.

Des moyens de mesure 6 sont prévus, tel un (ou des) capteur, lesquels sont aptes à et conçus pour mesurer au moins un paramètre représentatif du flux de gaz choisi parmi la pression du gaz, le débit de gaz insufflé par le respirateur, le débit de gaz expiré par le patient 20 et la vitesse de rotation de la micro-soufflante 40, et à délivrer au moins un signal représentatif dudit au moins un paramètre mesuré.

Par exemple, le paramètre représentatif du flux de gaz est la pression du gaz dans le circuit de gaz ventilatoire 2 et les moyens de mesure 6 comprennent un capteur de pression dont la prise de pression est agencée dans ledit circuit ventilatoire 2 de manière à permettre une mesure de la pression gazeuse régnant dans tout ou partie du circuit ventilatoire 2.

Dans le premier mode de réalisation de la Figure 1, la prise de pression faisant office de moyens de mesure 6, est agencée hors de la carcasse 9 du ventilateur 1. Toutefois, elle peut également se trouver dans le ventilateur 1. D'ailleurs, dans le mode de réalisation de la Figure 2, la prise de pression faisant office de moyens de mesure 6 a été représentée dans la carcasse 9 du ventilateur 1.

Une fois la mesure du (ou des) paramètre(s) représentatif(s) du flux de gaz opérée, le (ou les) signal correspondant est transmis à et analysé par des moyens de traitement de signal et de pilotage 5, 8 qui peuvent alors en déduire :
- qu'un massage cardiaque est en cours de réalisation sur le patient 20 et surtout déterminer si la phase en cours est une phase de compression ou une phase de relâchement de la cage thoracique;
- le volume de gaz insufflé par le ventilateur 1 au patient 20, au cours des cycles de ventilation mécanique et pendant les phases de relâchement de la cage de thoracique. Les volumes de gaz insufflé peuvent être cumulés sur une période de temps donnée, par exemple 1 minute. Bien entendu, le cumul peut être opéré sur plus de 1 min ou, à l'inverse, sur moins de 1 min.
- le volume de gaz expiré par le patient 20, au cours des cycles de ventilation mécanique et pendant les phases de relâchement de la cage de thoracique. Là aussi, les volumes de gaz expiré peuvent être cumulés sur une période de temps donnée, par exemple 1 minute. Bien entendu, le cumul peut être opéré sur plus de 1 min ou, à l'inverse, sur moins de 1 min.

La transmission du (ou des) signal représentatif du flux de gaz est transmis par les moyens de mesure 6 aux moyens de traitement de signal et de pilotage 5, 8 via une connectique adaptée, c'est-à-dire des liaisons électriques, tel que câbles ou autres.

Par ailleurs, les moyens de traitement de signal et de pilotage 5, 8 sont aptes à et conçus pour :
i) traiter le signal correspondant au paramètre représentatif du flux de gaz et y détecter une ou plusieurs variations positives ou négatives supérieures à une ou plusieurs valeurs-seuils représentatives des phases de compression ou de relâchement de la cage thoracique au cours d'un massage cardiaque. Ces valeurs-seuils sont enregistrées dans une mémoire 12 de stockage, par exemple une mémoire flash. Ces valeurs-seuils peuvent être des valeurs numériques, des tableaux de valeurs, des courbes...
ii) intégrer sur le signal correspondant au paramètre représentatif du flux de gaz, le débit de gaz généré par le ventilateur 1 pendant les compressions thoraciques et les cycles générés par la machine.
iii) intégrer par rapport au temps, le signal correspondant au paramètre représentatif du flux de gaz, et le débit de gaz généré par le ventilateur 1 pendant les compressions thoraciques et les cycles générés par le ventilateur 1.
iv) intégrer par rapport au temps, le signal correspondant au paramètre représentatif du flux de gaz, et le débit de gaz expiré par le patient 20 pendant les compressions thoraciques et les cycles générés par le ventilateur 1.

Pour ce faire, les moyens de traitement de signal 5, 8 comprennent de préférence un microprocesseur 8 programmé notamment avec un (ou plusieurs) algorithme(s) de traitement, comme détaillé ci-après.

Le ventilateur 1 et ses composants requérant de l'énergie pour fonctionner sont alimentés, directement ou indirectement, en courant électrique issu d'une ou plusieurs batteries, rechargeables ou non, de l'alimentation électrique du véhicule de secours qu'il équipe ou du secteur électrique, donc à une tension pouvant aller jusqu'à environ 230 V. Si besoin est, il peut intégrer un convertisseur de courant conçu pour abaisser la tension d'alimentation à une tension d'utilisation de valeur inférieure.

En outre, une interface homme-machine ou IHM 7, tel un écran d'affichage, permet d'afficher et donc à l'utilisateur de visualiser les informations calculées par les moyens de traitement de signal et de pilotage 5, 8.

Sont également prévus, des moyens de réglage ou de sélection 11, par exemple des boutons poussoirs ou rotatifs, des curseurs, des touches d'activation ou de sélection, ou analogue permettant au personnel soignant d'indiquer au ventilateur 1, la réalisation d'un massage cardiaque et/ou de confirmer au ventilateur 1, la détection qui a été faite de la réalisation d'un massage cardiaque, d'indiquer au ventilateur le type d'interface avec le patient, par exemple masque, sonde d'intubation...

Ainsi, on prévoit au moins un moyen de réglage ou de sélection 11, activable par l'opérateur, permettant de sélectionner un mode ventilatoire donné spécifique à une réanimation cardio-pulmonaire parmi plusieurs modes ventilatoires mémorisés.

Ces moyens de réglage ou de sélection 11 permettent également de modifier, si besoin est, les paramètres de la ventilation mécanique proposés automatiquement par le ventilateur 1, voire même, selon le mode de réalisation considéré, de pouvoir indiquer au ventilateur 1 un changement de la nature du gaz mis en oeuvre, par exemple le passage d'air à un mélange air/oxygène ou un changement de teneur en oxygène d'un mélange air/oxygène.

Comme on le voit sur les Figures 1 et 2, au moins une partie du circuit de gaz 2, les moyens de traitement de signal 8 et la source de gaz 4 sont agencés dans un capotage ou une carcasse rigide 9 qui forme l'enveloppe externe de l'appareil 1. Cette carcasse 9 inclut ou porte en outre d'autres composants, tel que l'IHM 7, la (ou les) mémoire 12, les moyens de réglage et sélection 11...

Le circuit de gaz 2, 16 comprend soit une branche inspiratoire 2 unique, soit une branche inspiratoire 2 et une branche expiratoire 16 par exemple reliées l'une à l'autre via une pièce en Y.

La branche inspiratoire 2 comprend en fait deux portions distinctes, à savoir une portion interne 2a agencée dans la carcasse rigide 9, par exemple un conduit de gaz, et une portion externe 2b située hors de la carcasse rigide 9 incluant par exemple un tuyau flexible.

La portion interne 2a du circuit de gaz 2 est en communication fluidique avec la source de gaz 4, typiquement une micro-soufflante motorisée ayant une prise ou entrée d'air 4a communiquant avec l'atmosphère ambiante, de manière à alimenter ladite portion interne 2a avec de l'air, éventuellement enrichi en oxygène.

La micro-soufflante motorisée 4 est pilotée par des moyens de pilotage 15, par exemple une carte électronique à microprocesseur, tel un microcontrôleur, mettant en oeuvre un (ou des) algorithme(s) :
En fait, les moyens de pilotage et de traitement de signal 5, 8 sont configurés pour piloter la micro-soufflante motorisée 4 en fonction des signaux transmis par les moyens de traitement de signal 5, 8 de manière à notamment permettre une synchronisation de l'envoi de gaz au patient 20 et des massages cardiaques.

En d'autres termes, les moyens de pilotage et de traitement de signal 5, 8 sont configurés pour piloter la valve expiratoire 19 en fonction des signaux transmis par les moyens de traitement de signal 5, 8 de manière à notamment permettre une synchronisation de l'envoi de gaz au patient 20 et des massages cardiaques.

Par ailleurs, la portion externe 2b du circuit de gaz 2 située hors de la carcasse rigide 9 est, quant à elle, en communication fluidique avec, du côté amont, la portion interne 2a du circuit de gaz 2 et, du côté aval, avec l'interface respiratoire 3 de manière à assurer une continuité fluidique entre la source de gaz 4 et le patient 20, et permettre d'acheminer le gaz respiratoire, e.g. l'air provenant de la turbine, jusqu'aux voies aériennes dudit patient.

Ici, les moyens de mesure 6, typiquement un (ou plusieurs) capteur, sont agencés sur la portion externe 2b de la branche inspiratoire 2 du circuit de gaz 2, 16 située hors de la carcasse rigide 9 de manière à opérer les mesures désirées, par exemple de pression et/ou de débit, au sein de ladite portion externe 2b. Dans ce cas, la liaison entre les moyens de mesure 6 et les moyens de traitement 5, et donc le transfert des signaux de mesure, se fait au moyen de liaisons filaires par exemple.

Optionnellement, la carcasse 9 peut également comprendre au moins une poignée de portage 13 pour faciliter le transport de l'appareil 1 par l'utilisateur, ce qui est indispensable dans certaines situations d'urgence, et/ou un dispositif d'accrochage 14 permettant l'accroche de l'appareil 1 de ventilation à un support, par exemple une tringle au sein d'un véhicule d'urgence ou un barreau de lit ou de brancard.

Par ailleurs, la branche expiratoire 16 comprend un capteur de débit expiratoire 17, par exemple un capteur à fil chaud, relié électriquement aux moyens de traitement de signal et de pilotage 5, 8, ainsi qu'une valve expiratoire 17 pilotée par les moyens de traitement de signal et de pilotage 5, 8. La branche expiratoire 16 communique, à son extrémité aval, avec l'atmosphère via un orifice de sortie de gaz 18, alors que son extrémité amont est reliée à la branche inspiratoire 2, via une pièce en Y, soit directement à l'interface patient 3.

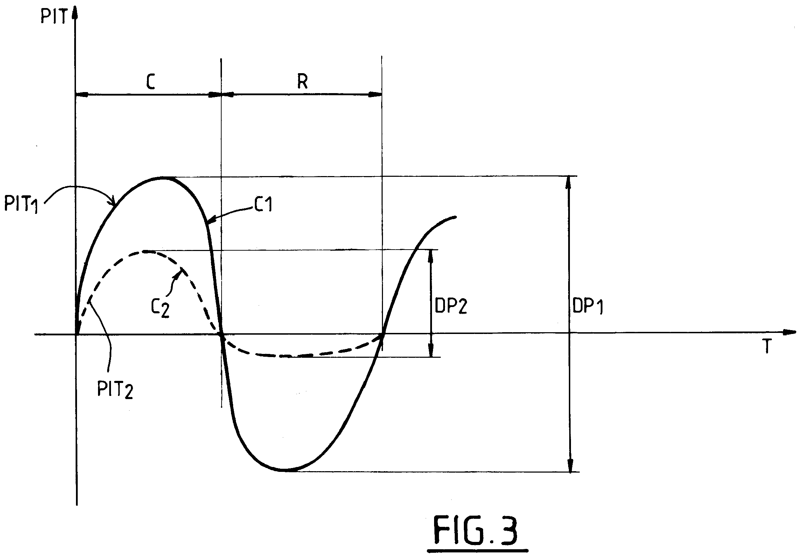

La Figure 3 est une représentation graphique illustrative de la pression intra-thoracique obtenue grâce à un appareil d'assistance respiratoire selon la présente invention.

Un massage cardiaque peut se décomposer comme étant une alternance et succession de phase de compression C et de phase de relâchement R (i.e. décompression) durant lesquelles la pression intra-thoracique (PIT) varie au cours du temps (T) entre des valeurs maximales et minimales comme illustré par les courbes de la Figure 3.

Plus précisément, la pression intra-thoracique PIT1 obtenue grâce à un appareil d'assistance respiratoire selon la présente invention permet d'obtenir une variation de pression DP1 (courbe C1) supérieure à la variation de pression DP2 calculée à partir de la pression intra-thoracique PIT2 (courbe C2) générée par un appareil de ventilation classique.

Au cours de la phase de compression C de la cage thoracique, déterminée grâce aux moyens de traitement de signal et de pilotage 5 et 8, le ventilateur pilote grâce à ces mêmes moyens de traitement de signal et de pilotage 5 et 8, la micro soufflante motorisée 40 ou la valve inspiratoire 41 et la valve expiratoire 19 des Figures 1 et 2 de manière à freiner l'éjection du volume d'air contenu dans le système respiratoire et ainsi maximiser la pression intra-thoracique 3 pendant cette phase de compression C.

Par ailleurs, au cours de la phase de décompression/relâchement R de la cage thoracique, déterminée grâce aux moyens de traitement de signal et de pilotage 5 et 8, le ventilateur pilote grâce à ces mêmes moyens de traitement de signal et de pilotage 5 et 8, la micro-soufflante motorisée 40 ou la valve inspiratoire 41 et la valve expiratoire 19 des Figures 1 et 2 de manière à limiter l'apport de gaz au système respiratoire et ainsi favoriser un retour passif de la cage thoracique en position d'équilibre et provoquer une pression intra-thoracique 3 négative pendant cette phase de décompression/relâchement R.

Pendant le massage cardiaque l'appareil de la présente invention délivre donc une ventilation barométrique entre 2 niveaux de pression, à savoir une pression basse (PB) et une pression haute (PH), avec PB<PH. Le massage cardiaque se poursuit aussi bien pendant l'application de la pression basse que pendant l'application de la pression haute. Le pilotage spécifique des actionneurs, i.e. micro soufflante motorisée 40 ou valve inspiratoire 41 et la valve expiratoire 19 notamment, pendant les phases de compression C et de relâchement R de la cage thoracique sont réalisées aussi bien sur le niveau de pression bas PB que de pression haut PH.

Le mode barométrique assure donc une régulation de pression alternée entre les 2 niveaux de pression PH et PB avec, par exemple une pression basse PB de l'ordre de 5 cm d'H₂O, et une pression haute PH de l'ordre de 15 cm d'H₂O.

Ce mode de ventilation spécifique à la réanimation cardio-pulmonaire permet d'assurer la ventilation d'un patient en arrêt cardiaque, du début à la fin de l'intervention et ce, dans un environnement nécessitant peu ou pas d'interventions humaines, lors des différentes phases.

D'une façon générale, selon la présente invention, les moyens de traitement de signal et de pilotage 5, 8 sont configurés pour, c'est-à-dire conçus pour et aptes à, piloter la micro-soufflante motorisée 40 ou, selon le mode de réalisation considéré, la valve inspiratoire 41 de sorte d'ajuster le volume ou la pression de gaz fourni par la micro-soufflante motorisée 40 ou la valve inspiratoire 41 en fonction de la phase de massage cardiaque détectée, lors d'une réanimation cardio-pulmonaire (RCP).

Ainsi, en réponse à la détection d'une phase de compression, les moyens de traitement de signal et de pilotage 5, 8 de l'appareil de l'invention vont commander la micro-soufflante motorisée 40 ou la valve inspiratoire 41, de manière à obtenir augmentation du volume ou de la pression de gaz fourni par la micro-soufflante motorisée 40 ou la valve inspiratoire 41, alors qu'en réponse à la détection d'une phase de relâchement/décompression, lesdits moyens de traitement de signal et de pilotage 5, 8 vont commander la micro-soufflante motorisée 40 ou la valve inspiratoire 41, de manière à diminuer le volume ou la pression de gaz foumi par la micro-soufflante motorisée 40 ou la valve inspiratoire 41.

Un tel pilotage va alors permettre, grâce à la détection des phases de compression et de décompression thoraciques opérées pendant la réalisation d'un massage cardiaque sur le patient en arrêt cardiaque, de transmettre au coeur un maximum d'énergie mécanique engendrée par les compressions thoraciques en limitant l'énergie dissipée via les voies aériennes, y compris les poumons, en particulier grâce à un ajustement judicieux du volume et/ou de la pression de gaz fourni par la micro-soufflante motorisée 40 ou la valve inspiratoire 41 en fonction de la phase de massage cardiaque détectée.

L'appareil d'assistance respiratoire selon la présente invention est utilisable dans le cadre d'une ventilation d'une personne en arrêt cardiaque soumise à des massages cardiaques.

## Revendications

1. Appareil d'assistance respiratoire (1) comprenant :
- une micro-soufflante motorisée (40) ou un dispositif d'alimentation en gaz (41, 55) comprenant une valve inspiratoire (41),
- un circuit de gaz (2, 16) avec au moins une branche inspiratoire (2) apte à véhiculer un gaz respiratoire destiné à être administré à un patient en arrêt cardiaque pendant une réanimation cardio-pulmonaire,
- des moyens de mesure (6) aptes à et conçus pour :
i) mesurer au moins un paramètre représentatif dudit flux de gaz,
ii) convertir ledit au moins un paramètre représentatif dudit flux de gaz en au moins un signal représentatif dudit flux de gaz,
- et des moyens de traitement de signal et de pilotage (5, 8) aptes à et conçus pour :
a) traiter ledit au moins un signal représentatif du flux de gaz fourni par les moyens de mesure (6) et
b) déduire dudit au moins signal représentatif du flux de gaz, une information relative à la réalisation d'un massage cardiaque sur le patient en arrêt cardiaque,
**caractérisé en ce que** les moyens de traitement de signal et de pilotage (5, 8) sont configurés pour piloter la micro-soufflante motorisée (40) ou la valve inspiratoire (41) de manière à :
i) en réponse à la détection d'une phase de compression, augmenter le volume ou la pression de gaz fourni par la micro-soufflante motorisée (40) ou la valve inspiratoire (41), et
ii) en réponse à la détection d'une phase de relâchement/décompression, diminuer le volume ou la pression de gaz fourni par la micro-soufflante motorisée (40) ou la valve inspiratoire (41).

2. Appareil selon la revendication précédente, **caractérisé en ce que** les moyens de traitement de signal et de pilotage (5, 8) comprennent une carte électronique et sont aptes à et conçus pour déduire dudit au moins un signal représentatif du flux de gaz, au moins une information relative à au moins une phase de compression et/ou de relâchement/décompression de la cage thoracique lors d'un massage cardiaque opéré sur le patient.

3. Appareil selon l'une des revendications précédentes, **caractérisé en ce que** la source de gaz (4) est une micro-soufflante motorisée pilotée par les moyens de traitement de signal et de pilotage, ladite micro-soufflante motorisée étant en communication fluidique avec la branche inspiratoire (2) du circuit de gaz (2, 16)

4. Appareil selon l'une des revendications 1 ou 2, **caractérisé en ce que** le dispositif d'alimentation en gaz (41, 55) comprend une valve inspiratoire (41) agencée sur un conduit de gaz interne (55), ladite valve inspiratoire étant pilotée par les moyens de traitement de signal et de pilotage (5, 8).

5. Appareil selon l'une des revendications précédentes, **caractérisé en ce qu'**il comprend au moins un moyen de sélection (11), activable par l'opérateur, permettant de sélectionner un mode ventilatoire donné spécifique à une réanimation cardio-pulmonaire parmi plusieurs modes ventilatoires mémorisés.

6. Appareil selon l'une des revendications précédentes, **caractérisé en ce que** les moyens de mesure (6) conçus pour et aptes à mesurer au moins un paramètre représentatif du flux de gaz, ledit paramètre représentatif du flux de gaz étant choisi parmi la pression du gaz, le débit de gaz insufflé au patient, le débit de gaz expiré par le patient et la vitesse de la micro-soufflante (4).

7. Appareil selon l'une des revendications précédentes, **caractérisé en ce qu'**au moins une partie du circuit de gaz (2, 16), les moyens de traitement de signal (5, 8) et la micro-soufflante motorisée (40) ou la valve inspiratoire (41) du dispositif d'alimentation (41, 55) sont situés dans une carcasse rigide (9).

8. Appareil selon l'une des revendications précédentes, **caractérisé en ce que** le circuit de gaz (2, 16) comprend une portion interne (2a) agencée dans la carcasse rigide (9) et une portion externe (2b) située hors de la carcasse rigide (9) et formant tout ou partie de la branche inspiratoire (2).

9. Appareil selon l'une des revendications précédentes, **caractérisé en ce qu'**il comprend en outre une interface homme-machine (7) apte à afficher des informations incluant au moins une information relative à la réalisation d'un massage cardiaque sur le patient en arrêt cardiaque.

10. Appareil selon l'une des revendications précédentes, **caractérisé en ce que** le circuit de gaz (2, 16) comprend en outre une branche expiratoire (16) en communication fluidique avec l'atmosphère via un orifice de sortie de gaz (18), et comportant une valve expiratoire (19) et un capteur de débit expiratoire (17).

11. Appareil selon l'une des revendications précédentes, **caractérisé en ce que** la portion externe (2b) de la branche inspiratoire (2) du circuit de gaz (2, 16) est en communication fluidique avec une interface respiratoire (3), en particulier un masque respiratoire ou une canule d'intubation.

12. Appareil selon l'une des revendications précédentes, **caractérisé en ce que** les moyens de traitement de signal et de pilotage (5, 8) sont configurés pour piloter la micro-soufflante motorisée (4) ou la valve inspiratoire, et la valve expiratoire (19) en fonction de signaux reçus des moyens de mesure (6) et du capteur de débit expiratoire (17).

13. Appareil selon l'une des revendications précédentes, **caractérisé en ce que** les moyens de traitement de signal et de pilotage (5, 8) sont configurés pour piloter la valve expiratoire (19) de manière à limiter ou stopper le débit de gaz traversant la valve expiratoire (19) en réponse à la détection d'une phase de compression ou de relâchement/décompression.

14. Appareil selon l'une des revendications précédentes, **caractérisé en ce que** les moyens de traitement de signal et de pilotage (5, 8) comprennent au moins un microprocesseur mettant en oeuvre au moins un algorithme.

15. Appareil selon l'une des revendications précédentes, **caractérisé en ce qu'**il comprend des moyens de stockage de données configurés pour mémoriser plusieurs modes ventilatoires comprenant au moins un mode ventilatoire donné spécifique à une réanimation cardio-pulmonaire.

## Patentansprüche

1. Einrichtung zur Atmungsunterstützung (1), umfassend:
- ein motorisiertes Mikrogebläse (40) oder eine Gasversorgungsvorrichtung (41, 55), umfassend ein Einatmungsventil (41),
- einen Gaskreislauf (2, 16) mit mindestens einem Einatmungszweig (2), der imstande ist, ein Atemgas zu befördern, das dazu bestimmt ist, einem Patienten mit Herzstillstand während einer Herz-Lungen-Wiederbelebung verabreicht zu werden,
- Messmittel (6), die imstande und ausgelegt sind zum:
i) Messen mindestens eines Parameters, der für die Gasströmung bezeichnend ist,
ii) Umwandeln des mindestens einen Parameters, der für die Gasströmung bezeichnend ist, in mindestens ein Signal, das für die Gasströmung bezeichnend ist,
- und Signalbehandlungs- und Steuermittel (5, 8), die imstande und ausgelegt sind zum:
a) Verarbeiten des mindestens einen Signals, das für die Gasströmung bezeichnend ist, das von den Messmitteln (6) bereitgestellt wird, und
b) Ableiten von dem mindestens einen Signal, das für die Gasströmung bezeichnend ist, einer Information bezüglich der Ausführung einer Herzmassage an dem Patienten mit Herzstillstand,
**dadurch gekennzeichnet, dass** die Signalbehandlungs- und Steuermittel (5, 8) konfiguriert sind, um das motorisierte Mikrogebläse (40) oder das Einatmungsventil (41) so zu steuern, dass:
i) als Reaktion auf die Erfassung einer Verdichtungsphase das Volumen oder der Druck des Gases, das von dem motorisierten Mikrogebläse (40) oder dem Einatmungsventil (41) bereitgestellt wird, erhöht wird, und
ii) als Reaktion auf die Erfassung einer Entlastungs-/Dekompressionsphase das Volumen oder der Druck des Gases, das von dem motorisierten Mikrogebläse (40) oder dem Einatmungsventil (41) bereitgestellt wird, verringert wird.

2. Einrichtung nach dem vorstehenden Anspruch, **dadurch gekennzeichnet, dass** die Signalbehandlungs- und Steuermittel (5, 8) eine Leiterplatte umfassen und imstande und ausgelegt sind, um von dem mindestens einen Signal, das für die Gasströmung bezeichnend ist, mindestens eine Information bezüglich mindestens einer Verdichtungsphase und/oder Entlastungs-/Dekompressionsphase des Brustkorbs bei einer Herzmassage, die an dem Patienten durchgeführt wird, abzuleiten.

3. Einrichtung nach einem der vorstehenden Ansprüche, **dadurch gekennzeichnet, dass** die Gasquelle (4) ein motorisiertes Mikrogebläse ist, das von den Signalbehandlungs- und Steuermitteln gesteuert wird, wobei sich das motorisierte Mikrogebläse in fluider Kommunikation mit dem Einatmungszweig (2) des Gaskreislaufs (2, 16) befindet.

4. Einrichtung nach einem der Ansprüche 1 oder 2, **dadurch gekennzeichnet, dass** die Gasversorgungsvorrichtung (41, 55) ein Einatmungsventil (41) umfasst, das auf einem internen Gaskanal (55) angeordnet ist, wobei das Einatmungsventil von den Signalbehandlungs- und Steuermitteln (5, 8) gesteuert wird.

5. Einrichtung nach einem der vorstehenden Ansprüche, **dadurch gekennzeichnet, dass** sie mindestens ein Auswahlmittel (11) umfasst, das von dem Bediener betätigbar ist, wodurch das Auswählen eines gegebenen Lüftungsmodus, der einer Herz-Lungen-Wiederbelebung eigen ist, aus mehreren gespeicherten Lüftungsmodi ermöglicht wird.

6. Einrichtung nach einem der vorstehenden Ansprüche, **dadurch gekennzeichnet, dass** die Messmittel (6) ausgelegt und imstande sind, mindestens einen Parameter zu messen, der für die Gasströmung bezeichnend ist, wobei der Parameter, der für die Gasströmung bezeichnend ist, aus dem Gasdruck, dem dem Patienten eingeblasenen Gasdurchsatz, dem von dem Patienten ausgeatmeten Gasdurchsatz und der Geschwindigkeit des Mikrogebläses (4) ausgewählt wird.

7. Einrichtung nach einem der vorstehenden Ansprüche, **dadurch gekennzeichnet, dass** sich mindestens ein Teil des Gaskreislaufs (2, 16), die Signalbehandlungs- und Steuermittel (5, 8) und das motorisierte Mikrogebläse (40) oder das Einatmungsventil (41) der Versorgungsvorrichtung (41, 55) in einem starren Gehäuse (9) befinden.

8. Einrichtung nach einem der vorstehenden Ansprüche, **dadurch gekennzeichnet, dass** der Gaskreislauf (2, 16) einen internen Abschnitt (2a), der in dem starren Gehäuse (9) angeordnet ist, und einen externen Abschnitt (2b) umfasst, der sich außerhalb des starren Gehäuses (9) befindet und den ganzen oder einen Teil des Einatmungszweiges (2) bildet.

9. Einrichtung nach einem der vorstehenden Ansprüche, **dadurch gekennzeichnet, dass** sie ferner eine Mensch-Maschine-Schnittstelle (7) umfasst, die imstande ist, Informationen anzuzeigen, die mindestens eine Information bezüglich der Ausführung einer Herzmassage an dem Patienten mit Herzstillstand einschließen.

10. Einrichtung nach einem der vorstehenden Ansprüche, **dadurch gekennzeichnet, dass** der Gaskreislauf (2, 16) weiter einen Ausatmungszweig (16) umfasst, der sich über eine Gasausgangsöffnung (18) in fluider Kommunikation mit der Atmosphäre befindet und ein Ausatmungsventil (19) und einen Ausatmungsdurchsatzsensor (17) aufweist.

11. Einrichtung nach einem der vorstehenden Ansprüche, **dadurch gekennzeichnet, dass** sich der externe Abschnitt (2b) des Einatmungszweiges (2) des Gaskreislaufs (2, 16) in fluider Kommunikation mit einer Atmungsschnittstelle (3), insbesondere einer Atemmaske oder einer Intubationskanüle, befindet.

12. Einrichtung nach einem der vorstehenden Ansprüche, **dadurch gekennzeichnet, dass** die Signalbehandlungs- und Steuermittel (5, 8) konfiguriert sind, um das motorisierte Mikrogebläse (4) oder das Einatmungsventil und das Ausatmungsventil (19) in Abhängigkeit von Signalen zu steuern, die von den Messmitteln (6) und dem Ausatmungsdurchsatzsensor (17) empfangen werden.

13. Einrichtung nach einem der vorstehenden Ansprüche, **dadurch gekennzeichnet, dass** die Signalbehandlungs- und Steuermittel (5, 8) konfiguriert sind, um das Ausatmungsventil (19) so zu steuern, dass der Gasdurchsatz, der durch das Ausatmungsventil (19) fließt, als Reaktion auf die Erfassung einer Verdichtungsphase oder Entlastungs-/Dekompressionsphase begrenzt oder gestoppt wird.

14. Einrichtung nach einem der vorstehenden Ansprüche, **dadurch gekennzeichnet, dass** die Signalbehandlungs- und Steuermittel (5, 8) mindestens einen Mikroprozessor umfassen, der mindestens einen Algorithmus anwendet.

15. Einrichtung nach einem der vorstehenden Ansprüche, **dadurch gekennzeichnet, dass** sie Datenspeichermittel umfasst, die konfiguriert sind, um mehrere Lüftungsmodi zu speichern, die mindestens einen gegebenen Lüftungsmodus umfassen, der einer Herz-Lungen-Wiederbelebung eigen ist.

## Claims

1. Respiratory aid apparatus (1) comprising:
- a motorised micro-blower (40) or a gas supply device (41, 55) comprising an inhalation valve (41),
- a gas circuit (2, 16) with at least one inhalation limb (2) able to transport a respiratory gas to be administered to a patient in cardiac arrest during a cardiopulmonary resuscitation,
- measurement means (6) able and designed to:
i) measure at least one parameter representative of said gas flow,
ii) convert said at least one parameter representative of said gas flow into at least one signal representative of said gas flow,
- and signal processing and control means (5, 8) able and designed to:
a) process said at least one signal representative of the gas flow provided by the measurement means (6) and
b) deduce from said at least one signal representative of the gas flow, an information relating to a cardiac massage being performed on the patient in cardiac arrest,
**characterised in that** the signal processing and control means (5, 8) are configured to control the motorised micro-blower (40) or the inhalation valve (41) so as to:
i) in response to the detection of a compression phase, increase the volume or gas pressure provided by the motorised micro-blower (40) or the inhalation valve (41), and
ii) in response to the detection of a release/decompression phase, reduce the volume or gas pressure provided by the motorised micro-blower (40) or the inhalation valve (41).

2. Apparatus according to the preceding claim, **characterised in that** the signal processing and control means (5, 8) comprise a printed circuit board and are able and designed to deduce from said at least one signal representative of the gas flow, at least one information relating to at least one compression and/or release/decompression phase of the rib cage during a cardiac massage performed on the patient.

3. Apparatus according to any of the preceding claims, **characterised in that** the gas source (4) is a motorised micro-blower controlled by the signal processing and control means, said motorised micro-blower being in fluid communication with the inhalation limb (2) of the gas circuit (2, 16).

4. Apparatus according to any of the claims 1 or 2, **characterised in that** the gas supply device (41, 55) comprises an inhalation valve (41) arranged on an internal gas duct (55), said inhalation valve being controlled by the signal processing and control means (5, 8).

5. Apparatus according to any of the preceding claims, **characterised in that** it comprises at least one selection means (11), that can be activated by the operator, enabling to select a given ventilation mode specific to a cardiopulmonary resuscitation among several ventilation modes stored in a memory.

6. Apparatus according to any of the preceding claims, **characterised in that** the measurement means (6) designed and able to measure at least one parameter representative of the gas flow, said parameter representative of the gas flow being selected from the gas pressure, the flow rate of the gas administered to the patient, the flow rate of the gas breathed out by the patient, and the speed of the micro-blower (4).

7. Apparatus according to any of the preceding claims, **characterised in that** at least one part of the gas circuit (2, 16), the signal processing means (5, 8) and the motorised micro-blower (40) or the inhalation valve (41) of the supply device (41, 55) are located in a rigid casing (9).

8. Apparatus according to any of the preceding claims, **characterised in that** the gas circuit (2, 16) comprises an inner portion (2a) arranged in the rigid casing (9) and an outer portion (2b) located outside the rigid casing (9) and forming all or part of the inhalation limb (2).

9. Apparatus according to any of the preceding claims, **characterised in that** it further comprises a man-machine interface (7) able to display information, including at least one information relating to a cardiac massage being performed on the patient in cardiac arrest.

10. Apparatus according to any of the preceding claims, **characterised in that** the gas circuit (2, 16) further comprises an expiratory limb (16) in fluid communication with the atmosphere through a gas outlet orifice (18), and comprising an expiratory valve (19) and an expiratory flow sensor (17).

11. Apparatus according to any of the preceding claims, **characterised in that** the outer portion (2b) of the inhalation limb (2) of the gas circuit (2, 16) is in fluid communication with a respiratory interface (3), in particular a respirator or an intubation cannula.

12. Apparatus according to any of the preceding claims, **characterised in that** the signal processing and control means (5, 8) are configured to control the motorised micro-blower (4) or the inhalation valve and the expiratory valve (19) based on signals received from the measurements means (6) and from the expiratory flow sensor (17).

13. Apparatus according to any of the preceding claims, **characterised in that** the signal processing and control means (5, 8) are configured to control the expiratory valve (19) so as to limit or interrupt the gas flow through the expiratory valve (19) in response to the detection of a compression or release/decompression phase.

14. Apparatus according to any of the preceding claims, **characterised in that** the signal processing and control means (5, 8) comprise at least one microprocessor implementing at least one algorithm.

15. Apparatus according to any of the preceding claims, **characterised in that** it comprises data storage means configured to store several ventilation modes, comprising at least one given ventilation mode specific to a cardiopulmonary resuscitation.
